# EUROPEAN PATENT SPECIFICATION

(11) **EP 1 129 354 B1**
(45) Date of publication and mention of the grant of the patent: **06.08.2008**
(21) Application number: 99956348.9
(22) Date of filing: 15.11.1999
(51) Int. Cl.: G01N 33/68, C07K 1/04, C12Q 1/68

(54) **A METHOD FOR DETERMINING A MIMOTOPE SEQUENCE**
VERFAHREN ZUR BESTIMMUNG EINER MIMOTOP-SEQUENZ
METHODE DE DETERMINATION D'UNE SEQUENCE DE MIMOTOPE

(30) Priority: 13.11.1998 EP 98203830
(43) Date of publication of application: 05.09.2001
(73) Proprietor: PEPSCAN SYSTEMS B.V., 8219 PH Lelystad (NL)
(72) Inventor: SLOOTSTRA, Jelle, Wouter, NL-8232 AJ Lelystad (NL); PUIJK, Wouter, Cornelis, NL-8243 VZ Lelystad (NL)
(74) Representative: Prins, Adrianus Willem
(86) International application number: PCT/NL1999/000700
(87) International publication number: WO 2000/029851

(56) References cited:
- WO-A-94/26775
- US-A- 5 556 762
- US-A- 5 565 325
- US-A- 5 582 997
- US-A- 5 645 996
- US-A- 5 763 193
- US-A- 5 814 470
- HALE G: "Synthetic peptide mimotope of the CAMPATH-1 (CD52) antigen, a small glycosylphosphatidylinositol-anchored glycoprotein" IMMUNOTECHNOLOGY, vol. 1, no. 3, 1 December 1995 (1995-12-01), page 175-187 XP004052720
- DE KOSTER H S ET AL: "The use of dedicated peptide libraries permits the discovery of high affinity binding peptides" JOURNAL OF IMMUNOLOGICAL METHODS, vol. 187, no. 1, 16 November 1995 (1995-11-16), page 179-188 XP004020980

## Description

The invention relates to a method for determining a mimotope sequence.

Nowadays, random diversity libraries are widely used to identify lead molecules for diagnostics, pharmaca and vaccins. When the lead molecule is a peptide, the methods used for identifying the lead molecules are often referred to as pepscan methods.

Pepscan methods have been known since the early eighties. The basic theory behind these methods is described in EP-A-0 138 855 and EP-A-0 190 205.

Two pivotal aspects of screening of random diversity libraries are their size, i.e. how many different compounds are required to identify a lead molecule, and the method of optimization of the structure or sequence of the lead molecule. Most methods described in the literature seem to be based on the idea that the larger the random diversity library, the higher the chance of finding a good lead molecule.

The aspect with respect to which the known methods are most divergent, is the method of optimization of the lead molecule. Once the random diversity library has been synthesized, it can be tested for its desired activity. Of course it is possible to simply choose as a lead molecule the member of the random diversity library that scores the highest value for said desired activity. However, most methods comprise steps for optimizing the structure of said member that shows the highest activity, in order to arrive at a lead molecule that shows an even higher activity.

Minipepscan libraries, composed of only a few thousand peptides, have been used to identify lead molecules which were quickly optimized to molecules having an activity similar to that of molecules arrived at from starting from libraries composed of millions of compounds. This has been disclosed in Slootstra et al., Molecular Diversity, 1 (1995b) 87-96, and in Slootstra et al., J. Mol. Recogn., 10, 217-224 Other background art is for instance described in US 5 814 474 A, US 5 645 996 A, US 5 582 997 A, US 5 565 325 A, US 5 556 762 A, Immunotechnology (1995) 1 175-187 and J. Immunol. Mtds (1995) 187 179-188. (1997). In these articles, it has been shown that small libraries in combination with pepscan-based optimization methods are a valuable tool in identifying and optimizing lead molecules.

The present invention aims to provide an improved method for identifying and optimizing a lead molecule.

It has been found that the objective improvement is achieved by starting from a library of known test molecules, evaluating the activity of said library, and by optimizing the structure of a few of test molecules showing the highest activity, by methodically replacing each building block of the structure of the test molecules with all other possible building blocks.

Thus, the invention relates to a method for determining a mimotope sequence for a receptor comprising the steps of:
a) providing a library of test sequences composed of building blocks chosen from the group of amino acids, monosaccharides and nucleotides
b) determining the activity of each test sequence of the library towards the receptor;
c) identifying a test sequence comprising at a certain position a building block which, according to the results of step b), shows a binding activity towards the receptor;
d) providing a next library of test sequences, based on said test sequence identified in step c), by replacing a building block at selected positions of the identified test sequence with selected building blocks;
e) determining the activity of each test sequence of the library provided in step d) towards the receptor;
f) identifying a test sequence comprising at a certain position a building block which, according to the results of step e), shows a binding activity toward the receptor;
g) repeating steps d) - f) for the library of test sequences provided in step d), for a number of cycles until in step f) a mimotope sequence that gives a threshold activity towards the receptor whereby the amount of receptor used for determining the activity of the test sequences toward said receptor is lowered in each cycle; wherein each test sequence is located on a minicard or flat support medium.

It has been found that the present method leads, in a convenient manner, to a mimotope sequence having a very high binding strength to the desired receptor. In some cases, where the exact epitope (i.e. the partial sequence of an antigen, that provides binding to an antibody) is known, the present method has been found to lead to the exact structure of the epitope in only a few steps.

According to the invention, a mimotope sequence for a receptor is determined. In this context, a mimotope sequence is defined as a molecule that shows a certain, minimal, desired activity in the presence of a given receptor. An example hereof is the determination of the epitope sequence of an antigen for a certain antibody. However, in some cases it may be sufficient if the exact epitope is mimicked and that a slightly less binding strength of the found molecule to the receptor suffices.

The mimotope sequence will be a molecule that is composed of a number of building blocks, wherein the number and order of building blocks controls the properties of the molecule. Examples of types of mimotope sequences that may be determined according to the invention include peptides, possibly having steroid or saccharide-like structures connected thereto, saccharides, DNA (oligonucleotides), and PNA (peptide-like nucleic acids). Thus, the building blocks are chosen from the groups of amino acids (both natural and non-natural amino acids), monosaccharides, and nucleotides.

The receptor for which a mimotope sequences is determined according to the invention may be any compound, composition, microorganism, or tissue sample towards which one of the types of mimotope sequences may show some activity that can be measured, such as a binding activity. Suitable examples of receptors include antibodies (both monoclonal and polyclonal), proteins, such as enzymes, cells, hormone receptors, and micro-organisms.

The first step in a method according to the invention is the provision of a library of test sequences. Of course, these test sequences are composed of the same type or class of building blocks as the objective mimotope sequence. Preferably, the test sequences are known in that, although they may be randomly chosen, their composition and structure is known. This may be accomplished by generating, for instance by hand or by computer, a number of sequences and synthesizing the sequences thus obtained. It is also possible to derive the library of test sequences from a compound which is known to have a favorable activity towards the receptor. In principle, any known manner of selecting test sequences to make up the library is suitable.

The length of the test sequences is dependent on the nature of the building blocks constituting the sequences, and on the nature of the receptor and the desired activity towards said receptor. For most purposes, a length of between 3 and 20 building blocks will be suitable.

The number of test sequences constituting the library will be large enough to provide sufficient data to come to a good mimotope sequence in an acceptable number of steps/cycles. On the other hand, said number will be small enough to assure that the data obtained can be handled quite conveniently. Usually, the number of test sequences in the library will lie between 500 and 100,000, preferably between 1,000 and 10,000.

After the desired number of test sequences is generated, said test sequences may be synthesized. This may be done in any known manner, for instance as has been described by Slootstra et al. in Molecular Diversity, 1 (1995b), 87-96. Of course, it is also possible to make use of a library which is already available, for instance because it has been used in a previous run of the present method. In that case, the test sequences will not have to be synthesized.

The test sequences are synthesized using a minicard or a flat support medium.

The next step of a method according to the invention is the determination of the activity of each test sequence of the library towards the receptor. The manner wherein this determination is carried out will depend on the specific interaction between mimotope sequence and receptor that is aimed at, and on the nature of the receptor and the building blocks of the mimotope sequence. For instance, when the desired activity is a binding of the mimotope sequence to the receptor, and the mimotope sequence is a peptide and the receptor is a monoclonal antibody, the determination may suitable be performed in an ELISA test, either in solution or on a solid support. Other suitable methods of determining the activity include BIACORE and AFM (Atomic Force Microscope). The skilled person will be able to choose a suitable manner of determination of the activity, given a certain receptor and nature of the mimotope sequence.

From the results of the determination of the activities of the test sequences of the library, at least one test sequence will be chosen to form a basis for the remaining steps of the present method. Said at least one test sequence is, in accordance with the invention, chosen by identifying at a certain position a building block which, as appears from the results of the determination of the activity, is favored at said position. In this regard, by the phrase "at a certain position a building block which is favored at said position" is meant that test sequences having at said position said building block show a high activity towards the receptor, relative to other activities found.

It is possible that the at least one test sequence which is identified was tested for its activity towards the receptor and showed itself a high activity, relative to other activities found.

It is also possible that the at least one test sequence which is identified, was in itself not tested. This can be explained as follows..For instance, it may have been found that a test sequence having at position 2 building block A shows a very high activity towards the receptor. It may further have been found that a test sequence having at position 4 building block B also shows a very high activity towards the receptor. The at least one test sequence to be identified may then be a sequence comprising building block A at position 2 and building block B at position 4. However, it may very well be that this sequence itself had not yet been evaluated for its activity towards the receptor.

Generally, the number of test sequences identified for basing the remaining steps of a method according to the invention on will be between 1 and 150. A higher number of test sequences chosen will lead to better results, but will be relatively more cumbersome to handle. Preferably, said number will be between 1 and.25. The skilled person will be able to decide on the number of test sequences chosen, dependent on the desired quality of the result of the method and the facilities available for carrying out the method.

On the basis of the identified test sequences, a next library of test sequences is provided in a so-called replacement analysis. This is done by varying the building blocks at positions of the identified test sequences, i.e. by replacing a building block at selected positions of the identified test sequence with selected building blocks.

For instance, in its simplest form the replacement analysis is carried out as follows. In case the test sequences are dodecapeptides of natural amino acids, first, the building block at position one of one of the chosen dodecapeptides may changed into each of the twenty naturally occurring amino acids, leading to 20 new test sequences. This is repeated for each position of the dodecapeptide, leading to 20 * 12 = 240 test sequences. Among these 240 sequences, the original dodecapeptide will be present 12 times. This procedure is repeated for each chosen dodecapeptide. Thus, a next library is obtained which comprises from 240 to 3600 new test sequences.

More complex forms of this replacement analysis may be performed, for instance by allowing certain building blocks to be replaced by groups of building blocks (e.g. the replacement of one amino acid by two or more amino acids). Usually, such a replacement by multiple building blocks will be used sparingly. Preferably, in at least one replacement analysis of the different cycles of the present method, at least one building block is replaced by a group of building blocks. Care should be taken that in each replacement analysis not too many multiple building blocks are introduced, for then problems concerning the length of the sequence may arise. For the same reason, the multiple group of building blocks should preferably not be too long. The skilled person will, based on his experience in the field, be able to judge which size and number of multiple building blocks may be introduced.

It is also possible to replace a building block by a void in the replacement analysis. That way, the sequence will become one building block shorter. It will be clear that not too many building blocks should be replaced by a void at the same time, as this could lead to an undesirably short (or no) test sequence. The skilled person will be able to judge when and at which position it is helpful to introduce a void into a test sequence.

In addition, it is possible to allow the introduction of selected building blocks in the replacement analysis only, or to only replace building blocks at selected positions in the test sequences. Sometimes it is preferred to maintain certain building blocks at certain positions, e.g. in order to maintain a desired three dimensional structure of the test sequence. An advantage of this manner of performing the replacement analysis is that the total number of test sequences to be provided is limited. Generally, however, if the building blocks at all positions of the test sequence are replaced by all possible building blocks, the test sequences having the desired three dimensional structure will also be provided. Again, it is within the skill of the artisan to judge in which cases it may be helpful to carry out a replacement at selected positions only. He will also be able to judge to which positions this may apply, and which building blocks may be used for the replacement and which building blocks may best not be used in the replacement (at certain positions).

The test sequences of the next library, which provided on the basis of the replacement analysis of the at least one identified test sequence, are tested for their activity towards the receptor. In case a test sequence is present among the test sequences of this library that shows sufficient activity towards the receptor, the method is completed and the desired mimotope sequence is obtained. It will be clear that it will depend on the type of activity, the receptor, the mimotope sequence, and the objective application of the mimotope sequence, whether a certain activity is regarded sufficient. Given the circumstances, the skilled person will be able to choose a threshold for a desired activity.

In case none of the test sequences shows sufficient activity towards the receptor, another cycle is carried out. In accordance with the invention, preferably at least two cycles, and thus two replacement analyses, are carried out. Thus, a next library of test sequences is provided, based on the 10 to 15 test sequences of the previous library that showed the highest activity, as set forth above. The test sequences in this next library will be evaluated for their activity, and so on.

The number of cycles that has to be carried out will depend on the desired degree of activity of the mimotope sequence to be found for the receptor. Starting from about 4500 randomly chosen dodecapeptides, it has been found possible to arrive at the exact epitope sequence for a monoclonal antibody in less than three cycles.

In a preferred embodiment of the invention, the amount of receptor used for determining the activity of the test sequences towards said receptor, is lowered in each cycle. It has been found that building blocks that seem essential in the first cycle may become non-essential in further cycles. In other words, in said first cycle local optima may be found, which may be overcome in a subsequent cycle or in subsequent cycles. The lowering of the amount of the receptor in the determining of the activity of the test sequences has been found to assist in overcoming such local optima. Preferably, the amount of receptor used in the determination of the activities of test sequences in a library is reduced by a factor of from 50 to 1000, more preferably from 10 to 100.

The invention will now be further elucidated by the following, non-restrictive examples.

### EXAMPLES

The described examples illustrate six variations of the present optimization method that has been successfully applied to lead peptides, identified with antibodies (monoclonal and polyclonal) raised against peptides, proteins, viruses, bacteria, sugars and steroids, from various types of peptide libraries. It can be envisaged that additional variations are possible. The concept that rules these variations is the repetition of replacement analyses on 1 or more lead peptides. Local minima are overcome and epitopes and mimotopes are identified.

### Materials and Methods

### Synthesis and screening minicards libraries (used in examples 1, 2, 5, 6)

Using the 20 natural L-amino acids 4550 random dodecapeptides (12-mers) were generated with a random generator programmed in Quick basic which runs on a 486 DX2 (66 MHz) computer system. In this library the frequency of each residue is approximately 5%. This set of sequences was used to design additional libraries. One in which all sequences are in the D-amino acid form, one in which the 2nd and 11th position are held by a cysteine and one in which the 3rd and 10th position are held by a cysteine. The aim of the latter two is to introduce a disulphide bridge into the peptide which should present the peptides as loops. Very important here is that the sequence of the amino acids within and outside the cysteines is identical to that of the first set of 4550 dodecapeptides. In this way the activity of these sets of dodecapeptides can be compared. **Example-1** describes results obtained with the synthetic peptide library one in which the 3rd and 10th position are held by a cysteine.
**Example-2** describes results obtained with the synthetic peptide library composed of 4550 random dodecapeptides (12-mers), i.e. no cysteine or any other motif was used.

The libraries were synthesized and screened using credit-card format mini-PEPSCAN cards (455 peptides/card) as described previously (Slootstra et al., 1995b). In **example-1** the binding of monoclonal antibody 26/9 to each peptide was tested in a PEPSCAN-based enzyme-linked immuno assay (ELISA). Monoclonal antibody 26/9 has been described previously (Wilson et al., 1984; Rini et al., 1992; Churchill et al., 1994). Monoclonal antibody 26/9 was raised against the peptide HA175-110 of the hemagglutinin protein of influenza virus (X47:HA1) (Wilson et al., 1984).

In **example-1** the 455-well creditcard-format polyethylene cards, containing the covalently linked peptides, were incubated with antibody 26/9 (100 *µ*g/ml). After washing the peptides were incubated with rabbit-antimouse peroxidase (rampo, dilution 1/1000) (Dakopatts) (1 hr, 25oC), and subsequently, after washing the peroxidase substrate 2,2'-azino-di-3-ethylbenzthiazoline sulfonate (ABTS) and 2 *µ*l/ml 3% H2O2 were added. After 1 hr the color development was measured. The color development of the ELISA was quantified with a CCD-camera and an image processing system. The setup consists of a CCD-camera and a 55 mm lens (Sony CCD Video Camara XC-77RR, Nikon micro-nikkor 55 mm f/2.8 lens), a camara adaptor (Sony Camara adaptor DC-77RR) and the Image Processing Software package TIM, version 3.36 (Difa Measuring Systems, The Netherlands). TIM runs on a 486 DX2 (50 MHz) computer system.

The dodecapeptide library, composed of 4550 random dodecapeptides, is screened with relatively high concentration of antibody (100 *µ*g/ml, more usually 10 *µ*g/ml, see **examples 2-6**). This concentration is approximately 2 orders of magnitude above the concentration required to obtain maximal binding activity in ELISA with the native epitope peptide (not shown). The concentration of 100 *µ*g/ml was used to obtain many binding peptides from the small set of 4550 random dodecapeptides. In previous studies it was shown that such a strategy, without obtaining an unfavorable signal to noise ratio, can result in hundreds of binding peptides that resemble small linear or non-linear parts of the native epitope (Slootstra et al., 1995b; Slootstra et al., 1997).

### Synthesis and screening of OTHER pepscan libraries (used in example 4)

In addition to random minipepscan libraries mimotopes can also be identified from standard pepscan libraries. These libraries contain all overlapping 12-mers (or shorter/longer) covering the linear sequence of a known protein (Slootstra et al., 1995a).

### Synthesis and screening of non-pepscan libraries (used in examples 3, 6)

Lead peptides can also be derived from other type of libraries such as for example phage-display libraries.

### Sequence analysis of lead molecules (used in example-5)

All 4550 dodecapeptides were ranked according to their binding activity. According to their ranking consensus sequences and motifs are identified. Initial methods of lead optimization that only use the sequence of the top 50 molecules have been described in Slootstra et al.(1995b; 1997). The present method uses all 4550 sequences. Firstly, the frequency and/or distribution of single amino acids and the dipeptide motifs OO, dipeptide motifs OXO and dipeptide motifs OXXO is determined using Microsoft Excel 4.0 (O, one of the 20 natural L-amino acids; X, any residue). Secondly, properties of the amino acids (hydrophobicity, charge etc.) are included in the analysis. Thirdly, all this data is used to optimize the activity of the top 10-50 lead molecules. This is done by substitution of building blocks that inhibit activity and include building blocks that improve binding activity.

The present sequence analysis method improves the activity of lead molecules through motif analysis of all sequences part of the library.

### Replacement analyses (used in examples 1, 2, 3, 4, 5, 6)

In a replacement analysis the binding activity of complete series of substitutions analogs of lead peptides, in which each position is replaced by each of the other 19 natural L-amino acids, is investigated in detail. In addition, it is possible to use the 20 D-amino acids as well as other available non-natural amino acids. Substitutions that result in improved binding are combined into new sequences of building blocks. These new sequences are again tested in a 2nd replacement analysis. If necessary more rounds of replacement analyses are performed. After each round of a replacement analyses the antibody (or other soluble receptor) concentration can be lowered to obtain maximal binding activity (e.g. from 100 *µ*g/ml to 0.01 *µ*g/ml after 2 rounds of replacement-analyses, as shown in **example-**1).

### References

- Churchill M.E.A., Stura E.A., Pinilla C., Appel J.R., Houghten R.A., Kono D.H., Balderas R.S., Fieser G.G., Schulze-Gahmen U. and Wilson I.A. (1994). Crystal structure of a peptide complex of anti-influenza peptide antibody Fab 26/9. J. Mol. Biol. 241, 534-556.
- Rini J.M., Schulze-Gahmen U. and Wilson I.A. (1992). Structural evidence for induced fit as a mechanism for antibody-antigen interaction. Science, 255, 959-965.
- Slootstra J.W., De Geus P., Haas H. Verrips, C.T. and Meloen R.H. (1995a). Possible active site of the sweet-tasting protein thaumatin. Chemical Senses, 20, 535-543.
- Slootstra J.W., Puijk W.C. Ligtvoet G.J., Langeveld J.P.M. and Meloen R.H. (1995b). Structural aspects of antibody-antigen interaction revealed through small random peptide libraries. Molecular Diversity 1, 87-96.
- Slootstra J.W., Kuperus D., Plückthun, A. and Meloen R.H. (1996). Identification of new tag sequences with differential and selective recognition properties for the anti-FLAG monoclonal antibodies M1, M2 and M5. Molecular Diversity 2, 156-164.
- Slootstra J.W., Puijk W.C. Ligtvoet G.J., Kuperus D., W.M.M. Schaaper and Meloen R.H. (1997). Screening of a small set of random peptides: A new strategy to identify peptides that mimic epitopes. J. Mol. Recog. 10, 219-224.
- Wilson I.A., Niman H.L., Houghten R.A., Cherenson A.R., Connolly M.L. and Lerner R.A. (1984). The structure of an antigenic determinant in a protein. Cell, 37, 767-778.

**Notes for the examples:**
- the 10 *µ*g/ml, 1 *µ*g/ml etc. denote the antibody concentration.
- The line "improved position-01" etc. means that this change in amino acid (is underlined) improves binding activity in pepscan elisa at given antibody concentration. Positions that could not be improved are not mentioned (e.g. position-08 in first replacement-net of example-1, i.e. the original I at position 8 gives the highest activity).

### Example-1:

title: Identification of an epitope through a lead peptide selected from 4550 random dodecapeptides (minipepscan library with motif XXCXXXXXXCXX, X , randomly selected amino acid, cf. Materials and Method))
tools: lead peptide CGCAAMNIRCYA
**methodology:** two rounds of replacement analyses

### Results:

In example-1, the lead peptide CGCAAMNIRCYA was derived (with antibody 26/9) from the random library in which the 3rd and 10th position are held by a cysteine (see above in Materials and methods). All possible single substitution analogs of the random peptide CGCAAMNIRCYA tested in pepscan for binding antibody 26/9 at 100 *µ*g/ml. Below these results are described in detail.

The replacement analysis of the lead peptide CGCAAMNIRCYA resulted in the identification of building blocks that cannot be replaced by any other building blocks (e.g. Y and A), and to the identification of residues that can be replaced by one or two other building blocks (e.g. N), and to residues that can be replaced by many other building blocks (e.g. M). Some replacements improve binding activity considerably (e.g. N into D).

All these replacements were used to design the improved peptide EMDEEEDIMNYA. Note that this peptide binds antibody 26/9 at much lower concentration, i.e. has improved binding activity (100 *µ*g/ml for CGCAAMNIRCYA and 1.0 *µ*g/ml for EMDEEEDIMNYA).

The peptide EMDEEEDIMNYA was run through a second replacement analysis. Again some replacements improve binding activity considerably. These replacements were used to design the improved peptide EMDEEEDVPDYA. Essential is that the first part of EMDEEEDIMNYA, EMDEEE, does not contain critical residues whereas the latter part, DIMNYA, does. Combination of the improved residues in this latter part results in the sequence DVPDYA. The sequence DVPDYA is identical to the linear epitope of antibody 26/9. Thus, the lead peptide CGCAAMNIRCYA derived from a few thousand random dodecapeptides was turned into native epitope sequence through two replacement analyses.

| REPLACEMENT ANALYSIS-I | |
|---|---|
| original lead: | CGCAAMNIRCYA |
| | activity at ≥ 100.0 *µ*g/ml |
| improved position-01: | EGCAAMNIRCYA |
| | activity at ≥ 10.0 *µ*g/ml |
| improved position-02: | CMCAAMNIRCYA |
| | activity at ≥ 10.0 *µ*g/ml |
| improved position-03: | CGDAAMNIRCYA |
| | activity at ≥ 10.0 *µ*g/ml |
| improved position-04: | CGCEAMMIRCYA |
| | activity at ≥ 10.0 *µ*g/ml |
| improved position-05: | CGCAEMNIRCYA |
| | activity at ≥ 10.0 *µ*g/ml |
| improved position-06: | CGCAAENIRCYA |
| | activity at ≥ 10.0 µg/ml |
| improved position-07: | CGCAAMDIRCYA |
| | activity at ≥ 10.0 *µ*g/ml |
| improved position-09: | CGCAANINIMCYA |
| | activity at ≥ 10.0 *µ*g/ml |
| improved position-10: | CGCAAMNIRNYA |
| | activity at ≥ 10.0 *µ*g/ml |
| combination improvements: | EMDEEEDIMNYA |
| | activity at ≥ 1.0 *µ*g/ml |

| REPLACEMENT ANALYSIS-II | |
|---|---|
| combinations rep-an. I: | EMDEEEDIMNYA |
| | activity at ≥ 1.0 *µ*g/ml |
| improved position-08: | EMDEEEDVMNYA |
| | activity at ≥ 0.1 *µ*g/ml |
| improved position-09: | EMDEEEDIPNYA |
| | activity at ≥ 0.1 *µ*g/ml |
| improved position-10: | EMDEEEDIMDYA |
| | activity at ≥ 0.1 *µ*g/ml |
| combination improvements: | EMDEEEDVPDYA |
| | activity at ≥ 0.01 *µ*g/ml |

| | |
|---|---|
| Notes: The sequence DVPDYA is the original epitope. The peptide EMDEEEDVPDYA has a 10-fold improved binding affinity (in solution) over the native epitope peptide YPYDVPDYASLRS. | |

### Example-2:

title: Identification of a mimotope through a lead peptide selected from 4550 random dodecapeptides (random minipepscan library)
**tools:** lead peptide ANWPSAIGAFGL
**methodology:** three rounds of replacement analyses

### Results:

In example-2, lead peptides were identified through a random minipepscan library. The difference of example-2 with example-1 is that the antibody used in example-1 binds a linear epitope whereas the antibody used in example-2 binds a non-linear epitope. The following multiple replacement analyses were done as discussed in example-1. The identified mimotope does not resemble any region of the linear sequence of the native protein.

| REPLACEMENT ANALYSIS-I | |
|---|---|
| original lead: | ANWPSAIGAFGL |
| | activity at ≥ 10.0 *µ*g/ml |
| improved position-01: | HNWPSAIGAFGL |
| | activity at ≥ 5.0 *µ*g/ml |
| improved position-02: | AWWPSAIGAFGL |
| | activity at ≥ 5.0 *µ*g/ml |
| improved position-03: | ANAPSAIGAFGL |
| | activity at ≥ 5.0 *µ*g/ml |
| improved position-04: | ANWSSAIGAFGL |
| | activity at ≥ 5.0 *µ*g/ml |
| improved position-11: | ANWSSAIGAFKL |
| | activity at ≥ 5.0 *µ*g/ml |
| combination improvements: | HWASSAIGAFKL |
| | activity at ≥ 1.0 *µ*g/ml |

| REPLACEMENT ANALYSIS-II | |
|---|---|
| combinations rep-an. I: | HWASSAIGAFKL |
| | activity at ≥ 1.0 *µ*g/ml |
| improved position-01: | KWASSAIGAFKL |
| | activity at ≥ 0.5 *µ*g/ml |
| improved position-02: | HYASSAIGAFKL |
| | activity at ≥ 0.5 *µ*g/ml |
| improved position-03: | HWGSSAIGAFKL |
| | activity at ≥ 0.5 *µ*g/ml |
| improved position-07: | HWASSAMGAFKL |
| | activity at ≥ 0.5 *µ*g/ml |
| combination improvements: | KYGSSAMGAFKL |
| | activity at ≥ 0.1 *µ*g/ml |

| REPLACEMENT ANALYSIS-III | |
|---|---|
| combinations rep-an. II: | KYGSSAMGAFKL |
| | activity at ≥ 0.1 *µ*g/ml |
| improved position-03: | KYFSSAMGAFKL |
| | activity at ≥ 0.05 *µ*g/ml |
| improved position-06: | KYGSSGMGAFKL |
| | activity at ≥ 0.05 *µ*g/ml |
| combination improvements: | KYFSSGMGAFKL |
| | activity at ≥ 0.01 *µ*g/ml |

### Example-3:

**title:** Identification of a mimotope through a lead derived from phage-display library composed of >1000.000 random hexapeptides.
**tools:** lead peptide SDTRKG*
**methodology:** lengthening to the left and to the right of SDTRKG with cysteines/glycines, followed by three rounds of replacement-analyses

### Results:

In example-3, lead peptides were identified through phage-display. Adjacent cysteines/glycines were added to improve binding activity. After obtained activity multiple replacement analyses were done as discussed in example-1.

| LENGTHENING BY CYSTEINES/GLYCINES: | |
|---|---|
| phage-display lead: | SDTRKG |
| | NO activity at 10.0 *µ*g/ml |
| lengthened lead: | CSDTRKGC |
| | activity at ≥ 10.0 *µ*g/ml |
| lengthened lead: | CSDTRKGCG |
| | activity at ≥10.0 *µ*g/ml |

| REPLACEMENT ANALYSIS-I | |
|---|---|
| lengthened lead: | CSDTRKGCG |
| | activity at ≥ 10.0 *µ*g/ml |
| improved position-02: | CTDTRKGCG |
| | activity at ≥ 5.0 *µ*g/ml |
| improved position-03: | CSETRKGCG |
| | activity at ≥ 5.0 *µ*g/ml |
| improved position-05: | CSDTHKGCG |
| | activity at ≥ 5.0 *µ*g/ml |
| improved position-06: | CSDTRYGCG |
| | activity at ≥ 5.0 *µ*g/ml |
| combination improv.: | CTETHYGCG |
| | activity at ≥ 1.0 *µ*g/ml |

| REPLACEMENT ANALYSIS-II | |
|---|---|
| comb. rep-an. I: | CTETHYGCG |
| | activity at ≥ 1.0 *µ*g/ml |
| improved position-02: | CYETHYGCG |
| | activity at ≥ 0.5 *µ*g/ml |
| improved position-05: | CTETKYGCG |
| | activity at ≥ 0.5 *µ*g/ml |
| improved position-06: | CTETHFGCG |
| | activity at ≥ 0.5 *µ*g/ml |
| combination improv.: | CYETKFGCG |
| | activity at ≥ 0.1 *µ*g/ml |

| REPLACEMENT ANALYSIS-III | |
|---|---|
| comb. rep-an. II: | CYETKFGCG |
| | activity at ≥ 0.1 *µ*g/ml |
| improved position-01: | DYETKFGCG |
| | activity at ≥ 0.05 *µ*g/ml |
| improved position-08: | CYETKFGNG |
| | activity at ≥ 0.05 *µ*g/ml |
| combination improv.: | DYETKFGNG |
| | activity at ≥ 0.01 *µ*g/ml |

| | |
|---|---|
| *, this lead peptide is, as a synthetic peptide, not active (not in elisa (pepscan or standard nor in solution). This is not unique. Often phage-peptides are only active as part of the phage-coat protein. In other formats they lose their activity. | |

### Example-4:

**title:** Identification of a mimotope through a lead derived from a standard pepscan analysis.

**tools:** lead peptide RVMIKLILVNFR* and complete sequence of native protein (part which was used is KIYRVMIKLILVNFRMQP).

**methodology:** Two rounds of replacement-analyses, followed by lengthening to the left and right, again followed by two more rounds of replacement-analyses, finally followed by lenthening to an 18-mer mimotope.

### Results:

In example-4, a lead peptide was identified through standard pepscan analysis, i.e. the antibody was tested on all overlapping 12-mers covering the linear sequence of the protein. The following two rounds of replacement analyses were done as discussed in example-1. In addition, the mimotope was lengthened to the left and right (3 amino acids, this can be further lengthened) through additional replacement analyses.

| REPLACEMENT ANALYSIS-I | |
|---|---|
| original lead: | RVMIKLILVNFR |
| | activity at ≥ 10.0 *µ*g/ml |
| improved position-01: | AVMIKLILVNFR |
| | activity at ≥ 5.0 *µ*g/ml |
| improved position-02: | AIMIKLILVNFR |
| | activity at ≥ 5.0 *µ*g/ml |
| improved position-03: | AVPIKLILVNFR |
| | activity at ≥ 5.0 *µ*g/ml |
| improved position-08: | AVMIKLIRVNFR |
| | activity at ≥ 5.0 *µ*g/ml |
| improved position-11: | AVMIKLILVNYR |
| | activity at ≥ 5.0 *µ*g/ml |
| combination improv.: | AIPIKLIRVNYR |
| | activity at ≥1.0 *µ*g/ml |

| REPLACEMENT ANALYSIS-II | |
|---|---|
| comb. rep-an. I: | AIPIKLIRVNYR |
| | activity at ≥ 1.0 *µ*g/ml |
| improved position-01: | YIPIKLIRVNYR |
| | activity at ≥ 0.5 *µ*g/ml |
| improved position-02: | APPIKLIRVNYR |
| | activity at ≥ 0.5 *µ*g/ml |
| combination improv.: | YPPIKLIRVNYR |
| | activity at ≥ 0.1 *µ*g/ml |

| LENTHENING WITH NATIVE SEQUENCE: | |
|---|---|
| comb. rep-an. II: | YPPIKLIRVNYR |
| | activity at ≥ 0.1 *µ*g/ml |
| lengthened left: | KIYYPPIKLIRV |
| | activity at ≥ 1.0 *µ*g/ml |
| lengthened right: | IKLIRVNYRMQP |
| | activity at ≥ 1.0 *µ*g/ml |

| REPLACEMENT ANALYSIS-III of left lengthened peptide: | |
|---|---|
| lengthened left: | KIYYPPIKLIRV |
| | activity at ≥ 1.0 *µ*g/ml |
| improved posit-01: | RIYYPPIKLIRV |
| | activity at ≥ 0.5 *µ*g/ml |
| improved posit-02: | KPYYPPIKLIRV |
| | activity at ≥ 0.5 *µ*g/ml |
| improved posit-03: | KIWYPPIKLIRV |
| | activity at ≥ 0.5 *µ*g/ml |
| improved posit-08: | KIYYPPISLIRV |
| | activity at ≥ 0.5 *µ*g/ml |
| combination impr.: | RPWYPPISLIRV |
| | activity at ≥ 0.1 *µ*g/ml |

| REPLACEMENT ANALYSIS-IV of right lengthened peptide: | |
|---|---|
| lengthened right: | IKLIRVNYRMQP |
| | activity at ≥ 1.0 *µ*g/ml |
| improved posit-10: | IKLIRVNYRCQP |
| | activity at ≥ 0.5 µg/ml |
| improved posit-11: | IKLIRVNYRMEP |
| | activity at ≥ 0.5 *µ*g/ml |
| improved posit-12: | IKLIRVNYRMQN |
| | activity at ≥ 0.5 *µ*g/ml |
| combination impr.: | IKLIRVNYRCEN |
| | activity at ≥ 0.1 *µ*g/ml |
| combination total: | RPWYPPISLIRVNYRCEN |
| | activity at ≥ 0.01 *µ*g/ml |

| | |
|---|---|
| *, lead peptide was identified from a library composed of all overlapping 12-mers covering the linear sequence of a protein. This makes it possible to lengthen the peptide to the left and right with adjacent amino acids, in this case KIY on the left and MQP on the right. Additional replacement-analyses of 12-mers, shifted three to the left or three to the right of the sequence finally results in an 18-mer mimotope. | |

### Example-5:

title: Identification of a mimotope through a set of similar leads selected from 4550 random dodecapeptides (random minipepscan library)
**tools:** set of 6 lead peptides QNNMKLFRGCVP, RGIKWNEMTDQW, KLQQNPTFYPPV, TNNCKEFAGIVP, RGILTNIMKDQW, IVQNNPKFFRGA (potentially up to all 4550 peptides, see material and method, in this case also 'negative' amino acids are removed from the leads)
**methodology:** : determination of consensus sequence, followed by two rounds of replacement analyses of the consensus sequence

### Results:

In example-5, a set of similar lead peptides were used to identify a consensus sequence. The consensus sequences were identified as discussed in materials and method. This sequence was used in the replacement analyses.
ALIGNMENT OF LEAD PEPTIDES:
QNNMKLFRGCVP
RGIKWNEMTDQW
KLQQNPTFYPPV
TNNCKEFAGIVP
RGILTNIMKDQW
IVQNNPKFFRGA

| consensus: ILONNMKDFRG | |
|---|---|
| REPLACEMENT ANALYSIS-I | |
| consensus lead: | ILQNNMKDFRG |
| | activity at ≥ 1.0 *µ*g/ml |
| improved position-03: | ILTNNMKDFRG |
| | activity at ≥ 0.5 *µ*g/ml |
| improved position-07: | ILQNNMPDFRG |
| | activity at ≥ 0.5 *µ*g/ml |
| improved position-10: | ILQNNMKDWRG |
| | activity at ≥ 0.5 *µ*g/ml |
| combination improv.: | ILTNNMPDWRG |
| | activity at ≥ 0.1 *µ*g/ml |

| REPLACEMENT ANALYSIS-II | |
|---|---|
| comb. rep-an. I: | ILTNNMPDWRG |
| | activity at ≥ 0.1 *µ*g/ml |
| improved position-07: | ILTNNMGDWRG |
| | activity at ≥ 0.05 *µ*g/ml |
| improved position-11: | ILTNNMPDWYG |
| | activity at ≥ 0.05 *µ*g/ml |
| combination improv.: | ILTNNMGDWYG |
| | activity at ≥ 0.01 *µ*g/ml |

### Example-6:

**title:** Identification of a mimotope through a set of different leads selected from phage-display library composed of >1,000,000 random hexapeptides.
**tools:** set of 3 lead peptides (ANWPSA, KLITRW, NVCSWS)
**methodology**: Two rounds of replacement analyses (for each lead peptide), followed by determination of overall consensus sequence.

### Results:

In example-6, different lead peptides were used to identify a consensus sequence. Each lead peptide was used in multiple replacement analyses (two rounds). The resulting three mimotopes were aligned which resulted in a lengthened mimotope with improved activity.

| REPLACEMENT ANALYSIS-IA | | | |
|---|---|---|---|
| original lead | : ANWPSA | _{:} activity at ≥ 10.0 | *µ*g/ml |
| improved position-01: | HNWPSA | : activity at ≥ 5.0 | *µ*g/ml |
| improved position-02: | AWWPSA | : activity at ≥ 5.0 | *µ*g/ml |
| improved position-03: | ANAPSA | : activity at ≥ 5.0 | *µ*g/ml |
| improved position-04: | ANWSSA | : activity at ≥ 5.0 | *µ*g/ml |
| combination improve. : | HWASSA | : activity at ≥ 1.0 | *µ*g/ml |

| REPLACEMENT ANALYSIS-IIA | | | |
|---|---|---|---|
| comb. rep-an. IA : | HWASSA | : activity at ≥1 .0 | *µ*g/ml |
| improved position-05: | HWASPA | : activity at ≥ 0.5 | *µ*g/ml |
| combination improve. : | HWASPA | : activity at ≥ 0.5 | *µ*g/ml |

| REPLACEMENT ANALYSIS-IB | | | |
|---|---|---|---|
| original lead : | KLITRW | : activity at ≥ 10.0 | *µ*g/ml |
| improved position-01: | SLITRW | : activity at ≥ 5.0 | *µ*g/ml |
| improved position-02: | KSITRW | : activity at ≥ 5.0 | *µ*g/ml |
| improved position-03: | KLATRW | : activity at ≥ 5.0 | *µ*g/ml |
| improved position-06: | KLITRY | : activity at ≥ 5.0 | *µ*g/ml |
| combination improve. : | SSATRY | : activity at ≥ 1.0 | *µ*g/ml |

| REPLACEMENT ANALYSIS-IIB | | | |
|---|---|---|---|
| comb. rep-an. IB : | SSATRY | : activity at ≥ 1.0 | *µ*g/ml |
| improved position-02: | SPATRY | : activity at ≥ 0.5 | *µ*g/ml |
| combination improv. : | SPATRY | : activity at ≥0.5 | *µ*g/ml |

| REPLACEMENT ANALYSIS-IC | | | |
|---|---|---|---|
| original lead | : NVCSWS | : activity at ≥ 10.0 | *µ*g/ml |
| improved position-02: | NICSWS | : activity at ≥ 5.0 | *µ*g/ml |
| improved position-04: | NVCHWS | : activity at ≥ 5.0 | *µ*g/ml |
| improved position-06: | NVCSWA | : activity at ≥ 5.0 | *µ*g/ml |
| combination improv. : | NICHWA | : activity at ≥ 1.0 | *µ*g/ml |

| REPLACEMENT ANALYSIS-IIC | | | |
|---|---|---|---|
| comb. rep-an. IC : | NICHWA | : activity at ≥ 1.0 | *µ*g/ml |
| improved position-01: | YICHWA | : activity at ≥0.5 | *µ*g/ml |
| improved position-02: | NVCHWA | : activity at ≥0.5 | *µ*g/ml |
| combination improv. : | YVCHWA | : activity at ≥ 0.1 | *µ*g/ml |
| ALIGNMENT combination | improv. | IIA, IIB and IIC: | |
| combination improvements 1: | | HWASPA | |
| combination improvements 2: | | SPATRY | |
| combination improvements 3: YVCHWA | | | |
| consensus: | YVCHWASSATRY | | |
| | activity at 0.01 *µ*g/ml | | |

## Claims

1. A method for determining a mimotope sequence for a receptor comprising the steps of:
a) providing a library of test sequences composed of building blocks chosen from the group of amino acids, monosaccharides, and nucleotides;
b) determining the activity of each test sequence of the library towards the receptor;
c) identifying a test sequence comprising at a certain position a building block which, according to the results of step b), shows a binding activity towards the receptor;
d) providing a next library of test sequences, based on said test sequence identified in step c), by replacing a building block at selected positions of the identified test sequence with selected building blocks;
e) determining the activity of each test sequence of the library provided in step d) towards the receptor;
f) identifying a test sequence comprising at a certain position a building block which, according to the results of step e), shows a binding activity towards the receptor;
g) repeating steps d) - f) for the library of test sequences provided in step d), for a number of cycles until in step f) a mimotope sequence is found that gives a threshold activity towards the receptor whereby the amount of receptor used for determining the activity of the test sequences towards said receptor is lowered in each cycle;
wherein each test sequence is located on a minicard or flat support medium.

2. A method according to claim 1, wherein the chemical composition of the test sequences provided in step a) is known.

3. A method according to claim 1 or 2, wherein in step e) the amount of receptor used for determining the activity is smaller by a factor in the range of from 50 to 1000 smaller than said amount used in step b), and wherein said amount in step g) is smaller by a factor in the range of from 50 to 1000 than said amount in step e) of the cycle directly preceding said step g).

4. A method according to claim 3, wherein in step e) the amount of receptor used for determining the activity is smaller by a factor in the range of from 10 to 100 smaller than said amount used in step b), and wherein said amount in step g) is smaller by a factor in the range of from 10 to 100 than said amount in step e) of the cycle directly preceding said step g).

5. A method according to any one of the preceding claims, comprising at least one step d) wherein at least one building block is replaced by a group of building blocks.

6. A method according to any one of the preceding claims, wherein the test sequences comprise from 3 to 20 building blocks.

7. A method according to any one of the preceding claims, wherein the library of test sequences of step a) comprises from 500 to 100,000 test sequences.

8. A method according to any one of the preceding claims, wherein the receptor is chosen from the group consisting of monoclonal antibodies, proteins, such as enzymes, cells, hormone receptors, and micro-organisms.

9. A method according to any one of the preceding claims, wherein the activity is determined using an immuno assay, BIACORE or AFM.

10. A method according to any one of the preceding claims, wherein each test sequence of a library is physically separated from the other test sequences of said library.

## Patentansprüche

1. Verfahren zur Bestimmung einer Mimotop-Sequenz für einen Rezeptor, das folgende Schritte umfasst:
a) Bereitstellen einer Bibliothek von Testsequenzen, die aus Baublöcken bestehen, welche aus der Gruppe der Aminosäuren, Monosacchariden und Nukleotiden ausgewählt werden;
b) Ermitteln der Aktivität jeder Testsequenz der Bibliothek gegenüber dem Rezeptor;
c) Identifizieren einer Testsequenz, die an einer bestimmten Position einen Baublock umfasst, der entsprechend den Ergebnissen aus Schritt b) eine Bindungsaktivität gegenüber dem Rezeptor zeigt;
d) Bereitstellen einer nächsten Bibliothek von Testsequenzen basierend auf der in Schritt c) identifizierten Testsequenz durch Ersetzen eines Baublocks an ausgewählten Positionen der identifizierten Testsequenz durch ausgewählte Baublöcke;
e) Ermitteln der Aktivität jeder Testsequenz der in Schritt d) bereitgestellten Bibliothek gegenüber dem Rezeptor;
f) Identifizieren einer Testsequenz, die an einer bestimmten Position einen Baublock umfasst, der entsprechend den Ergebnissen aus Schritt e) eine Bindungsaktivität gegenüber dem Rezeptor zeigt;
g) Wiederholen der Schritte d) - f) für die in Schritt d) bereitgestellte Bibliothek von Testsequenzen über eine Reihe von Zyklen, bis in Schritt f) eine Mimotop-Sequenz gefunden wird, bei der sich ein Schwellwert der Aktivität in Bezug auf den Rezeptor ergibt, wobei die Menge an Rezeptor, die zur Bestimmung der Aktivität der Testsequenzen gegenüber dem Rezeptor genutzt wird, in jedem Zyklus vermindert wird;
wobei jede Testsequenz auf einer Minikarte oder einem flachen Trägermedium angeordnet wird.

2. Verfahren nach Anspruch 1, bei welchem die chemische Zusammensetzung der in Schritt a) bereitgestellten Testsequenzen bekannt ist.

3. Verfahren nach Anspruch 1 oder 2, bei welchem in Schritt e) die Menge an Rezeptor, die zum Ermitteln der Aktivität genutzt wird, um einen Faktor im Bereich von 50 bis 1000 kleiner als die in Schritt b) genutzte Menge ist, und bei welchem die in Schritt g) genutzte Menge um einen Faktor im Bereich von 50 bis 1000 kleiner als die in Schritt e) des direkt diesem Schritt g) vorausgegangenen Zyklus genutzte Menge ist.

4. Verfahren nach Anspruch 3, bei welchem in Schritt e) die Menge an Rezeptor, die zum Ermitteln der Aktivität genutzt wird, um einen Faktor im Bereich von 10 bis 100 kleiner als die in Schritt b) genutzte Menge ist, und bei welchem die in Schritt g) genutzte Menge um einen Faktor im Bereich von 10 bis 100 kleiner als die in Schritt e) des direkt diesem Schritt g) vorausgegangenen Zyklus genutzte Menge ist.

5. Verfahren nach einem der vorhergehenden Ansprüche, welches zumindest einen Schritt d) umfasst, bei dem zumindest ein Baublock durch eine Gruppe von Baublöcken ersetzt wird.

6. Verfahren nach einem der vorhergehenden Ansprüche, bei welchem die Testsequenzen 3 bis 20 Baublöcke umfassen.

7. Verfahren nach einem der vorhergehenden Ansprüche, bei welchem die Bibliothek von Testsequenzen des Schrittes a) 500 bis 100.000 Testsequenzen umfasst.

8. Verfahren nach einem der vorhergehenden Ansprüche, bei welchem der Rezeptor aus der Gruppe bestehend aus monoklonalen Antikörpern, Proteinen, beispielsweise Enzymen, Zellen, Hormonrezeptoren und Mikroorganismen ausgewählt wird.

9. Verfahren nach einem der vorhergehenden Ansprüche, bei welchem die Aktivität mit Hilfe eines Immuntests, BIACORE oder AFM bestimmt wird.

10. Verfahren nach einem der vorhergehenden Ansprüche, bei welchem jede Testsequenz einer Bibliothek physikalisch von den anderen Testsequenzen dieser Bibliothek getrennt wird.

## Revendications

1. Procédé pour déterminer une séquence mimotope d'un récepteur comprenant les étapes de :
a) fournir une banque de séquences test composées de synthons choisis parmi le groupe d'acides aminés, de monosaccharides et de nucléotides ;
b) déterminer l'activité de chaque séquence test de la banque envers le précepteur ;
c) identifier une séquence test comprenant, à une certaine position, un synthon qui, selon les résultats de l'étape b), montre une activité de liaison envers le récepteur ;
d) fournir une banque suivante de séquences test, en se basant sur ladite séquence test identifiée dans l'étape c), en remplaçant un synthon en des positions choisies de la séquence test identifiée par des synthons choisis ;
e) déterminer l'activité de chaque séquence test de la banque fournie dans l'étape d) envers le récepteur ;
f) identifier une séquence test comprenant, à une certaine position, un synthon qui, selon les résultats de l'étape e), montre une activité de liaison envers le récepteur ;
g) répéter les étapes d)-f) pour la banque de séquences test fournie dans l'étape d), pour un nombre de cycles jusqu'à ce que dans l'étape f) une séquence mimotope soit trouvée qui donne une activité de seuil envers le récepteur grâce à quoi la quantité de récepteur utilisée pour déterminer l'activité des séquences test envers ledit récepteur est diminuée dans chaque cycle ;
dans lequel chaque séquence test est située sur un support plat ou une minicarte.

2. Procédé selon la revendication 1, dans lequel la composition chimique des séquences test prévues dans l'étape a) est connue.

3. Procédé selon la revendication 1 ou 2, dans lequel, dans l'étape e), la quantité de récepteur utilisée pour déterminer l'activité est plus faible d'un facteur dans la plage de 50 à 1000 que ladite quantité utilisée dans l'étape b), et dans lequel ladite quantité dans l'étape g) est plus faible d'un facteur dans la plage de 50 à 1000 que ladite quantité dans l'étape e) du cycle précédant directement ladite étape g).

4. Procédé selon la revendication 3, dans lequel, dans l'étape e), la quantité de récepteur utilisée pour déterminer l'activité est plus faible d'un facteur dans la plage de 10 à 100 que ladite quantité utilisée dans l'étape b), et dans lequel ladite quantité dans l'étape g) est plus faible d'un facteur dans la plage de 10 à 100 que ladite quantité dans l'étape e) du cycle précédant directement ladite étape g).

5. Procédé selon une quelconque des revendications précédentes, comprenant au moins une étape d) dans laquelle au moins un synthon est remplacé par un groupe de synthons.

6. Procédé selon une quelconque des revendications précédentes, dans lequel les séquences test comprennent de 3 à 20 synthons.

7. Procédé selon une quelconque des revendications précédentes, dans lequel la banque de séquences test de l'étape a) comprend de 500 à 100 000 séquences test.

8. Procédé selon une quelconque des revendications précédentes, dans lequel le récepteur est choisi à partir du groupe constitué d'anticorps monoclonaux, de protéines, telles que des enzymes, de cellules, de récepteurs hormonaux et de micro-organismes.

9. Procédé selon une quelconque des revendications précédentes, dans lequel l'activité est déterminée en utilisant un immunoessai, BIACORE ou un microscope à force atomique AFM.

10. Procédé selon une quelconque des revendications précédentes, dans lequel chaque séquence test d'une banque est séparée physiquement des autres séquences test de ladite banque.
